(19) Europäisches Patentamt
European Patent Office
Office européen des brevets

(11) EP 3 003 371 B1

# (12) EUROPEAN PATENT SPECIFICATION

(45) Date of publication and mention
of the grant of the patent:
**25.05.2022 Bulletin 2022/21**

(21) Application number: **14804544.6**

(22) Date of filing: **29.05.2014**

(51) International Patent Classification (IPC):
**A61K 39/395** *(2006.01)* **A61P 35/00** *(2006.01)*
**A61K 45/06** *(2006.01)* **A61K 35/17** *(2015.01)*
**C07K 16/22** *(2006.01)* **A61K 39/00** *(2006.01)*

(52) Cooperative Patent Classification (CPC):
(C-Sets available)
**C07K 16/22; A61K 35/17; A61K 45/06;**
**A61P 35/00;** A61K 2039/505; C07K 2317/732;
C07K 2317/76 (Cont.)

(86) International application number:
**PCT/CN2014/078740**

(87) International publication number:
**WO 2014/190914 (04.12.2014 Gazette 2014/49)**

## (54) MATERIALS AND METHODS FOR TREATMENT OF LIVER CANCER BACKGROUND OF THE INVENTION

MATERIALIEN UND VERFAHREN ZUR BEHANDLUNG VON LEBERKREBS

MATÉRIAUX ET MÉTHODES POUR LE TRAITEMENT DU CANCER DU FOIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.05.2013 US 201361829027 P**

(43) Date of publication of application:
**13.04.2016 Bulletin 2016/15**

(73) Proprietor: **The University of Hong Kong
Hong Kong (CN)**

(72) Inventors:
• **CHEUNG, Siu Tim**
**Hong Kong (CN)**
• **CHEUNG, Fung Yi**
**Hong Kong (CN)**
• **FAN, Sheung Tat**
**Hong Kong (CN)**

(74) Representative: **HGF
HGF Limited
1 City Walk
Leeds LS11 9DX (GB)**

(56) References cited:
WO-A1-2005/106019    WO-A1-2008/064570
WO-A1-2011/140828    WO-A2-98/19167
WO-A2-2005/009217    CN-A- 101 553 728
CN-A- 102 985 111    US-A1- 2008 199 470

• JENNY C. HO ET AL: "Granulin-epithelin precursor as a therapeutic target for hepatocellular carcinoma", HEPATOLOGY, vol. 47, no. 5, 1 May 2008 (2008-05-01), pages 1524-1532, XP055061009, ISSN: 0270-9139, DOI: 10.1002/hep.22191
• MASAHISA, J. ET AL.: 'Expression and role of MICA and MICB in human hepatocellular carcinomas and their regulation by retinoic acid.' INT. J. CANCER. vol. 104, 31 December 2003, pages 354 - 361, XP055234976
• JIAN-JUN LI ET AL: "Prognostic value of soluble MICA levels in the serum of patients with advanced hepatocellular carcinoma", AIZHENG - CHINESE JOURNAL OF CANCER, vol. 32, no. 3, 5 March 2013 (2013-03-05), pages 141-148, XP055525390, CN ISSN: 1000-467X, DOI: 10.5732/cjc.012.10025

- **KEISUKE KOHGA ET AL: "Serum levels of soluble major histocompatibility complex (MHC) class I-related chain A in patients with chronic liver diseases and changes during transcatheter arterial embolization for hepatocellular carcinoma", CANCER SCIENCE, vol. 99, no. 8, 1 August 2008 (2008-08-01) , pages 1643-1649, XP055654057, JP ISSN: 1347-9032, DOI: 10.1111/j.1349-7006.2008.00859.x**

(52) Cooperative Patent Classification (CPC): (Cont.)

C-Sets
**A61K 35/17;
A61K 38/18;
A61K 39/3955**

**Description**

BACKGROUND OF THE INVENTION

Liver cancer is the third leading cancer killer in the world, with more than half a million individuals dying globally each year. In China, liver cancer is the second major cause of cancer death. Surgical resection, in the form of a partial hepatectomy or a liver transplant, is the mainstay of curative treatment. Nonetheless, cancer recurrence is still common after curative surgery. In addition, liver cancer is frequently diagnosed at an advanced stage, which precludes curative treatment. No effective therapeutic option exists for the treatment of the majority of liver cancer patients.

Granulin-epithelin precursor (GEP) is a pluripotent growth factor regulating fetal development, tissue repair and tumorigenesis in various cancers. GEP is over-expressed in more than 70% of human hepatocellular carcinoma (HCC). GEP expression has been shown to associate with aggressive HCC features. Functional studies demonstrate that GEP plays a role in regulating HCC cell proliferation, invasion, tumorigenicity, and chemoresistance. Moreover, neutralization of GEP could inhibit the growth of established HCC in mouse model.

Cancer stem cells (CSCs) are considered as the "root" of cancers. CSCs are not only responsible for tumor initiation and progression, but also endowed with stem cell properties, including self-renewal, differentiation capacity and chemoresistance. Although existing therapies can initially eliminate the tumor bulk, stem cell properties of CSCs enable them to survive and repopulate the tumor, resulting in disease relapse. Thus, CSC eradication may be the key for curing aggressive malignancies including HCC.

The immune system has the ability to identify and eliminate tumor cells before they develop into malignancy. NK cells are the major component of the innate immune system and represent the first line of defense against tumors. Anti-tumor cytotoxicity of NK cells is mediated by direct lysis or induction of IFN-$\gamma$. However, it was reported that cytotoxicity of NK cells was impaired in HCC patients and the reduced activities of NK cells are associated with HCC progression.

NK cytotoxicity is regulated through integrated signaling from their cell surface receptors that interact with ligands expressed on target cells. NKG2D (natural killer group 2, member D) is a stimulatory receptor expressed on the surface of all NK cells and its recognition is crucial for tumor immunosurveillance. MHC class I chain-related molecule A (MICA), a ligand of human NKG2D receptor, is frequently expressed in tumors, but not in normal tissues. Engagement of MICA and NKG2D strongly activates NK cells, enhancing their cytotoxicity and cytokine production. Thus, the NKG2D-MICA pathway is an important mechanism by which host immune system recognizes and eliminates tumor cells. CD94/NKG2A is an inhibitory receptor that controls the activity of human NK cells following interactions with the non-classic class I human leukocyte antigen E (HLA-E) on target cells. HLA-E is ubiquitously expressed by nearly all cells in the body, but is over-expressed by tumor cells and is likely to protect the tumor cells from NK cytotoxic activity through inhibition via interactions with the CD94/NKG2A receptor.

Immune evasion is one of the important properties of CSCs that enable them to survive better in the host. Recently, ABCB5+ malignant melanoma initiating cells were found to possess the capacity to modulate anti-tumor immunity by preferentially inhibiting IL-2-dependent T-cell activation and to support induction of regulatory T cells. Moreover, CD200+ CSC cells were found to suppress anti-tumor immunity by down-regulating the expression of Thl cytokines and costimulatory molecules in ovarian cancer, melanoma and leukemia. However, evaluation of CSCs in the context of host immune responses has largely been disregarded in HCC. Therapeutic regimes that inhibit tumor cells from escaping from immunosurveillance can be used to treat hepatic CSCs.

Ho, J. C. et al., "Granulin-epithelin precursor as a therapeutic target for hepatocellular carcinoma", HEPATOLOGY, vol. 47, no. 5, pages 1524 - 1532, discloses granulin-epithelin precursor (GEP) as a target for treating hepatocellular carcinoma and that an anti-GEP monoclonal antibody is able to inhibit growth of established heptaocellular carcinoma tumours in a dose dependent manner.

WO2011/140828 (UNIV HONG KONG) discloses a composition comprising a granulin-epithelin precursor (GEP) antibody for treating hepatic cancer is disclosed. The composition further comprises a chemodrug and a drug transporter - specific antibody. GEP antibody for the treatment of hepatic cancer is also disclosed.

WO2008/064570 (UNIV HONG KONG) discloses methods for detecting serum GEP level and methods for determining whether a subject is afflicted with Hepatocellular carcinoma (HCC) by measuring serum GEP level. Also disclosed are methods for the suppression of HCC growth and progression both *in vitro* and *in vivo* by treating a patient with anti-GEP monoclonal antibody A23.

US2008/199470 (CHEUNG SIU TIM) discloses a method for detecting serum GEP level, and further, methods for determining whether a subject is afflicted with Hepatocellular carcinoma (HCC) by measuring serum GEP level. Also disclosed are methods for the suppression of HCC growth and progression both *in vitro* and *in vivo* by treating a patient with anti-GEP monoclonal antibody A23.

WO2005/009217 (MEDIMMUNE INC) discloses methods and compositions designed for the treatment or management of pre-cancerous conditions, especially in order to prevent, delay, or decrease the likelihood that the pre-cancerous condition will progress to malignant cancer. The methods comprise the administration of an effective amount of one or more agents that decrease/inhibit PCDGF expression, secretion, and/or activity. The pharmaceutical compositions comprise one or more PCDGF agents, and the PCDGF agents can be administered with other therapeutic agents for treatment or management of a pre-cancerous condition that are not PCDGFbased. Diagnostic methods and methods for screening for therapeutically useful PCDGF agents are also described.

WO2005106019A1 (UNIV HONG KONG) discloses methods for determining whether an agent causes a reduction in the activity of GEP in a cell. Also methods for reducing the expression of GEP protein in a cell. Also described are methods for determining whether a subject is afflicted with Hepatocellular carcinoma (HCC) and methods for treating them comprising administering a therapeutically effective amount of an agent which specifically interferes with the expression of GEP in tumour cells of the subject.

Masahisha, J., et al. (2003) "Expression and role of MICA and MICB in human hepatocellular carcinomas and their regulation by retinoic acid" Int. J. Cancer vol 104: pages 354 - 361 is a scientific publication about experiments in which retinoic acid-treated hepatoma cells induce IFN$\gamma$ production from cocultured NK cells and render themselves more susceptible to NK cells. An upregulation of MICA/B was observed and the results suggest that MICA/B, expressed on a subset of human HCCs, may play an important role in their susceptibility to NK cells. Also how retinoic acid can function as a modulator of MICA/B expression and thereby further activate NK cells.

## BRIEF SUMMARY OF THE INVENTION

The invention provides an anti-GEP antibody and (a) natural killer (NK) cells, or (b) an agent that promotes NK cell activity selected from IL-15, IL-12, pectin, interferon, IFN-gamma, IL-2 or IL-21, for use in treating liver cancer in a subject.

The invention further provides an anti-GEP antibody for use in preventing recurrence of or treating liver cancer in a subject, wherein the subject has had surgical resection of a primary liver tumor, has undergone hepatectomy, and/or had a liver transplant; and wherein (a) an effective amount of NK cells is also administered; or (b) an agent that promotes NK cell activity selected from IL-15, IL-12, pectin, interferon, IFN-gamma, IL-2 or IL-21 is also administered.

The invention also provides a GEP antisense polynucleotide and (a) natural killer (NK) cells, or (b) an agent that promotes NK cell activity selected from IL-15, IL-12, pectin, interferon, IFN-gamma, IL-2 or IL-21, for use in treating liver cancer in a subject.

The invention also provides a GEP antisense polynucleotide for use in preventing recurrence of or treating liver cancer in a subject, wherein the subject has had surgical resection of a primary liver tumor, has undergone hepatectomy, and/or has had a liver transplant; and wherein (a) an effective amount of NK cells is also administered; or (b) an agent that promotes NK cell activity selected from IL-15, IL-12, pectin, interferon, IFN-gamma, IL-2 or IL-21 is also administered.

The GEP antisense polynucleotide may be an siRNA.

Described herein is a method for suppressing immune evasion and for eradicating GEP-expressing CSC's in hepatocellular carcinoma (HCC). This is based on the surprising discovery that granulin-epithelin precursor (GEP) expression is associated with the immune evasion ability of human liver cancer, GEP is associated with the natural killer (NK) cell activity in human liver cancer, and anti-GEP antibody treatment enhances the NK cell cytotoxic activities against liver cancer cells.

## BRIEF DESCRIPTION OF THE DRAWINGS

The following explain the contents of the figures, although the figures are not present.

**Figure 1** shows that GEP expression of HCC cells modulates anti-tumor cytotoxic activity of immune cells. **(A)** GEP modulation in Hep3B (high endogenous GEP cell line) and HepG2 (low endogenous GEP cell line). Hep3B cells were stably transfected for GEP suppression by shRNA, while HepG2 cells were stably transfected for over-expression by full-length GEP cDNA. Protein expression of GEP in controls and transfectants was measured by flow cytometry. Grey line: isotype control staining. **(B)** GEP suppression increased, while over-expression decreased cell cytotoxicity of human PBMC against HCC cells. HCC cells (target cells) labeled with green fluorescence CFSE were co-cultured with human PBMC (effector cells) at effector/target ratio (E/T ratio) of 25 : 1 for 5 h. Cells were harvested and stained with propidium iodide (PI), with lysed HCC cells recognized as CFSE$^+$PI$^+$ and quantified by flow cytometry. Cell cytotoxicity level was calculated as follows: [cell cytotoxicity (%) = (Percentage of CFSE$^+$PI$^+$ cells / (Percentage of CFSE$^+$PI$^+$ cells plus percentage of CFSE$^+$PI$^-$ cells) x 100). *$P < 0.05$ when compared with control cells. **(C)** Depletion of NK cells markedly abolished the GEP-mediated cell cytotoxicity. CD56$^+$ NK cells were depleted from human PBMC by magnetic sorting. NK cells-depleted PBMC were then cultured with HCC cells for 5 hr at E/T ratio of 25:1. Cell cytotoxicity was then measured by flow cytometry. **(D)** GEP suppression increased, while over-expression decreased cell cytotoxicity of NK cells against HCC cells. NK cells were isolated from human PBMC using magnetic sorting and then cultured with HCC cells for 5 hours at indicated E/T ratio. * $P < 0.05$, ** $P <$

0.01 when compared with control cells at respective E/T ratio.

**Figure 2** shows that GEP differentially regulates the expression of MICA and HLA-E of HCC cells. **(A)** GEP suppression up-regulated membrane-bound MICA, and suppressed soluble MICA release and membrane-bound HLA-E expression. Hep3B cells (high endogenous GEP cell line) were stably transfected for GEP suppression by shRNA. Membrane-bound MICA and HLA-E expression was measured by surface immunofluorescence staining and flow cytometry. Gray line: isotype control staining. Soluble MICA in culture supernatant was measured by ELISA. **(B)** GEP over-expression down-regulated membrane-bound MICA, and increased soluble MICA production and membrane-bound HLA-E expression. HepG2 cells (low endogenous GEP cell line) were stably transfected for over-expression by full-length GEP cDNA. Gray line: isotype control staining.

**Figure 3** shows that GEP blockage by anti-GEP antibody A23 modulates the expression of MICA and HLA-E of HCC cells. Hep3B cells were treated with anti-GEP monoclonal antibody (A23), mouse IgG isotype antibody (IgG) (antibodies at 50 J.Lg/ml) or without antibody (control) for 24h. **(A)** A23 significantly reduced GEP expression of Hep3B cells. **(B)** A23 induced surface MICA expression, and suppressed soluble MICA release and surface HLA-E expression in Hep3B cells. Dotted line: isotype control staining. $*P< 0.05$, $**P < 0.01$ when compared with control.

**Figure 4** shows that GEP blockage by anti-GEP antibody A23 enhances NK cell cytotoxicity against HCC cells via MICA. (A) GEP blockage by anti-GEP antibody A23 enhances PBMC-mediated cytotoxicity against HCC cells. The cytotoxicity is suppressed by anti-MICA neutralizing antibody. Hep3B and HepG2 cells were treated with anti-GEP monoclonal antibody (A23), mouse IgG isotype control (migG) (50 J.Lg/ml) or without antibody (control) for 24h prior to co-culture with PBMC at E/T ratio of 25: 1 for 5h. For A23 treatment, cells were co-cultured with PBMC with or without neutralizing MICA antibody or mouse IgG2a isotype control (2 µg/ml) for 5h. $* P< 0.05$ when compared with control; $\#P < 0.05$ between groups denoted by horizontal lines. **(B)** A23-enhanced cell cytotoxicity of NK cells against Hep3B and HepG2 cells was suppressed by anti-MICA neutralizing antibody. $* P < 0.05$, $**P<0.0 1$, $** P < 0.001$ when compared with control; $\#P < 0.05$, $\#\# P < 0.001$ when compared with A23 alone treatment at respective E/T ratio.

**Figure 5** shows co-expression of GEP with **(A)** MICA and **(B)** HLA-E in HCC clinical specimens. Liver tissues were digested into disaggregated cell suspension, and assessed for the expression of GEP and MICA or HLA-E. Co-expression was performed by triple-color flow cytometry, gating on the albumin+ hepatocytes of HCC specimens. Protein expression of GEP was measured by intracellular staining, while those of membrane-bound MICA and HLA-E were assessed by surface staining. Cells co-expressing the respective markers were shown in the upper right quadrant of dot plots. Mean percentage of cells $\pm$ SD from 3 HCC clinical specimens were shown. Table summarizes the mean percentage of positive cells $\pm$ SD from 3 samples.

**Figure 6** shows antibody-dependent cellular cytotoxicity mediated by anti-GEP monoclonal antibody A23. A23 mediated ADCC against HCC cells dose-dependently. Hep3B or HepG2 cells labeled with CFSE were incubated with or without anti-GEP antibody A23 (A23) or mouse isotype control (migG) of the indicated concentrations of the antibodies for 30 min prior to co-culture with or without PBMC at E/T ratio of 25 : 1 for 5h. $* P < 0.05$, $** P < 0.01$ when compared with control cells without antibody. **(B)** Depletion of CD56+ NK cells from human PBMC markedly abolished A23-mediated ADCC. Hep3B or HepG2 cells labeled with CFSE were incubated with or without anti-GEP antibody A23 of indicated concentrations for 30 min prior to co-culture with PBMC or NK cell-depleted PBMC at E/T ratio of 25:1 for 5h. $* P<0.05$, $** P < 0.01$ when compared with control cells without antibody. (C) A23-mediated ADCC against HCC cells was dependent on NK cells. NK cells were isolated and cultured with HCC cells for 5 hr at indicated E/T ratio. $*P< 0.05$, $**P < 0.01$, $***P<0.001$ when compared with control cells without antibody.

**Figure 7** shows the effect of Natural Killer (NK) cells isolated from liver cancer patients against liver cancer cells. A. NK cells showed enhanced cytotoxicity in liver cancer cells with GEP suppression (hep3B cells transfected with GEP shRNA, 3B-sh1). B. NK cells showed decreased cytoxicity in liver cancer cells with GEP over-expression (HepG2 transfected with GEP full length, G2-FL).

BRIEF DESCRIPTION OF THE SEQUENCES

The following provides nucleotide and amino acid sequence information although the individual sequences are not actually shown herein.

**SEQ ID NO:1** is a cDNA sequence of a granulin-epithelin precursor (GEP).

**SEQ ID NO:2** is the amino acid sequence of a granulin-epithelin precursor.

**SEQ ID NO:3** is the amino acid sequence of part of GEP (GEP amino acids 578-593).

**SEQ ID NO:3a** is the nucleic acid sequence encoding amino acids 578-593 of GEP.

**SEQ ID NO:4** is the amino acid sequence of part of GEP (GEP amino acids 351-365).

**SEQ ID NO:4a** is the nucleic acid sequence encoding amino acids 351-365 of GEP.

**SEQ ID NO:5** is the amino acid sequence of part of GEP (GEP amino acids 574-593).

**SEQ ID NO: 5a** is the nucleic acid sequence encoding amino acids 574-593 of GEP.

**SEQ ID NO:6** is the amino acid sequence of part of GEP (GEP amino acids 337-363).

**SEQ ID NO:6a** is the nucleic acid sequence encoding amino acids 337-363 of GEP.

**SEQ ID NO:7** is the amino acid sequence of part of GEP signaling peptide (GEP amino acids 1-17).

**SEQ ID NO:7a** is the nucleic acid sequence encoding amino acids 1-17 of GEP.

**SEQ ID NO:8** is the amino acid sequence of part of the GEP linker (GEP amino acids 18-57).

**SEQ ID NO: 8a** is the nucleic acid sequence encoding amino acids 18-57 of GEP.

**SEQ ID NO:9** is the amino acid sequence of part of the GEP linker (GEP amino acids 114-122).

**SEQ ID NO: 9a** is the nucleic acid sequence encoding amino acids 114-122 of GEP.

**SEQ ID NO:10** is the amino acid sequence of part of the GEP linker (GEP amino acids 180-205).

**SEQ ID NO:10a** is the nucleic acid sequence encoding amino acids 180-205 of GEP.

**SEQ ID NO:11** is the amino acid sequence of part of the GEP linker (GEP amino acids 262-280).

**SEQ ID NO:11a** is the nucleic acid sequence encoding amino acids 262-280 of GEP.

**SEQ ID NO:12** is the amino acid sequence of part of the GEP linker (GEP amino acids 418-441).

**SEQ ID NO: 12a** is the nucleic acid sequence encoding amino acids 418-441 of GEP.

**SEQ ID NO:13** is the amino acid sequence of part of the GEP linker (GEP amino acids 497-517).

**SEQ ID NO: 13a** is the nucleic acid sequence encoding amino acids 497-517 of GEP.

DETAILED DISCLOSURE OF THE INVENTION

Described herein is a method for suppressing immune evasion and for eradicating the GEP-expressing CSCs in hepatocellular carcinoma (HCC). This is based on the surprising discovery that granulin-epithelin precursor (GEP) expression is associated with the immune evasion ability of human liver cancer; GEP is associated with the natural killer (NK) cell activity in human liver cancer; and anti-GEP antibody treatment enhances the NK cell cytotoxic activities against liver cancer cells.

Described herein is also the surprising discovery that serum GEP and MICA levels are elevated in subjects with HCC, and high levels of soluble GEP and/or soluble MICA are associated with poor recurrence-free survival. The inventors have shown that GEP down regulates MICA on the HCC cell surface, thereby rendering the HCC cells less susceptible to NK cell cytotoxicity.

In addition, the present inventors have shown soluble GEP levels to correlate with tumour size.

Effect of GEP on Immune Evasion and Cancer Stem Cell Population

Described herein is how granulin-epithelin precursor (GEP) rendered hepatocellular carcinoma (HCC) cells resistant to cytotoxic activity of NK cells, an important mechanism of anti-tumor responses. The alteration in immune response induced by GEP would cause an imbalance in the host's immune responses, allowing the subsequent growth of a tumor. Thus, GEP provides a selective advantage to cancer stem cells (CSCs) by enabling them to proliferate in hosts.

GEP blockage by monoclonal antibody A23 significantly suppresses GEP level in HCC cells, and the GEP-mediated regulation on MHC class I chain-related molecule A (MICA) and HLA-E expression on HCC cells could be reversed. As a result, the HCC cells can be sensitized to NK cytotoxic activity. In addition, anti-GEP monoclonal antibody A23 induces NK cell-mediated ADCC against HCC cells, and therefore further amplifies the anti-tumor response of the antibody. Therefore, targeting antibody that binds specific to GEP (such as A23 antibody) can be used for suppressing immune evasion and eradicating the GEP-expressing CSCs in HCC.

GEP defines a hepatic CSC population with greater chemoresistance, self-renewal and tumor-initiating ability. The present inventors discovered that GEP conferred the CSCs' ability to escape from NK immunosurveillance, giving a selective advantage to the GEP$^+$ cells and enabling them to survive and proliferate in the host.

As a hallmark of tumor progression and recurrence, tumors have developed diverse mechanisms to evade the immune system. It is postulated that tumor cells have evolved to down-regulate their surface MICA by proteolytic shedding, therefore reducing their susceptibility to NKG2D-mediated NK cytotoxicity. Also, the soluble MICA (sMICA) released from tumor cell surface can not only block the NKG2D-binding site for other NKG2D ligands, but also suppress NKG2D expression on NK cells. Production of sMICA therefore represents one of the mechanisms in tumor immune evasion. It was reported that sMICA were frequently elevated in advanced HCC patients and such elevation was associated with down-regulated NKG2D expression and impaired NK cell activation in HCC. Significance of sMICA as predictive and prognostic marker has been reported in HBV-induced HCC and advanced HCC.

GEP suppression is significantly up-regulated, while GEP over-expression reduced surface MICA level on HCC cells. GEP-expressing cells might therefore evade NK cytotoxicity by reducing surface MICA level and become less susceptible to NK cell responses. In addition, sMICA level decreased significantly in the culture supernatant of GEP-suppressed HCC cells, but increased significantly when GEP was over-expressed. The results show that GEP could confer HCC cells immune evasion ability by promoting shedding of MICA from cell surface and promote the release of sMICA which disturbs the NKG2D-mediated NK cytotoxicity.

Up-regulation of HLA-E, a ligand for the NK inhibitory immunoreceptor CD94/NKG2A9, has been suggested to represent a mechanism of tumor escape from NK cell immunosurveillance in ovarian cancer and melanoma. It was previously reported that HLA-E mRNA was up-regulated in HCC specimens in comparison with their adjacent normal counterpart. Also, HLA-E polymorphism was also associated with risk of hepatitis B or HCC. Nevertheless, before the present invention, the role of HLA-E in HCC pathogenesis has not been reported. In addition, it was not elucidated that HCC cells over-express HLA-E.

GEP expression up-regulates HCC cells' surface expression of HLA-E, which interacts with NK cell inhibitory immunoreceptor CD94/NKG2A and suppresses NK cell activity.

HLA-E could play a role in HCC pathogenesis by promoting the immune evasion ability of GEP-expressing CSCs. GEP blockage using anti-GEP monoclonal antibody A23 could significantly suppress GEP level and sensitize the GEP$^+$ cells to NK cytotoxic activity. The results show that antibodies that bind to GEP have antitumor effect against HCC cells once NK cells are efficiently activated during A23 treatment. The combination therapy of anti-HCC molecular targeted therapy and immunotherapy targeting activation of NK cells could improve the antitumor effect against unresectable HCC and the prognosis of patients with HCC. Also, as the present inventors have shown that GEP expression would induce shedding of MICA from HCC cell surface and increase soluble MICA, patients with higher soluble MICA level in their serum would be more responsive to the anti-GEP monoclonal antibody treatment. Criteria can be defined and used to select patients who are potentially responsive to anti- GEP antibody treatment.

In addition to immune escape, another immunologic mechanism known to play a role in the anti-tumor activity of antibodies is their ability to induce antibody-dependent cell-mediated cytotoxic activity (ADCC) mediated by cytotoxic immune cells (such as NK cells) to lyse the tumor cells. ADCC is an innate immune effector mechanism in which antibodies evoke tumor cell death when antibodies bind to antigen on tumor cells and the antibody Fc domains engage Fc receptors on the surface of immune effector cells.

Anti-GEP monoclonal antibody A23 may induce NK cell-mediated ADCC against HCC cells. Therefore, the anti-tumor effect of anti-GEP monoclonal antibody A23 can be further amplified by inducing destruction of the A23-coated HCC cells by NK cells. Targeting GEP by antibody A23 represents a novel therapeutic tool for suppressing immune evasion and for eradicating the GEP-expressing CSCs in HCC.

Liver Cancer or Tumor Therapy

In a specific embodiment of the invention of claim 2 or claim 4, the GEP antisense polynucleotide is administered to a liver tumor of the subject.

In embodiments of the invention, the anti-GEP antibody or the GEP antisense the agent that inhibits the expression or activity of granulin-epithelin polynucleotide may be administered before, during, or after NK cells and/or an agent that promotes NK cell activity. In another embodiment, the anti-GEP antibody or the GEP antisense polynucleotide and/or an agent that promotes NK cell activity is administered as a therapeutic composition.

Described herein is a method of treating liver cancer in a subject, the method comprising administering to a subject an effective amount of an agent that increases the activity or expression of MICA present on a liver cancer cell.

Also described herein is a method of treating liver cancer in a subject with increased levels of soluble MICA compared to a subject without liver cancer, the method comprising administering to the subject an effective amount of an agent that inhibits the expression or activity of granulin-epithelin precursor (GEP).

Additionally described is a method of increasing MICA expression on a cancer cell comprising administering an effective amount of an agent that inhibits the expression or activity of granulin-epithelin precursor (GEP).

The term "treatment" or any grammatical variation thereof (e.g., treat, treating, and treatment etc), as used herein, includes but is not limited to, ameliorating or alleviating a symptom of a disease or condition, reducing, suppressing, inhibiting, lessening, or affecting the progression, severity, and/or scope of a condition.

The term "prevention" or any grammatical variation thereof (e.g., prevent, preventing and prevention etc.), as used herein, includes but is not limited to, reducing the likelihood of developing a disease, delaying the onset of symptoms, preventing relapse to a disease, increasing latency between symptomatic episodes, or a combination thereof. Prevention, as used herein, does not require the complete absence of symptoms.

The term "effective amount," as used herein, refers to an amount that is capable of treating or ameliorating a disease or condition or otherwise capable of producing an intended therapeutic effect.

The term "subject," as used herein, describes an organism, including mammals such as primates, to which treatment with the compositions according to the subject invention can be provided. Mammalian species that can benefit from the disclosed methods of treatment include, but are not limited to, apes, chimpanzees, orangutans, humans, monkeys; and other animals such as dogs, cats, horses, cattle, pigs, sheep, goats, chickens, mice, rats, guinea pigs, and hamsters. In one embodiment, the subject has, or is diagnosed with, liver tumor or cancer. In one embodiment, the subject had underwent partial or total hepatectomy and/or liver transplantation.

The term "partial hepatectomy," refers to the surgical resection of less than the entire, but more than 5% (including, any percent higher than 5%, including but not limited to, more than 10%, 15%, 20%, 25%, 30%, 35%, 40%, 45%, 50%, 55%, 60%, 65%, 70%, 75%, 80%, 85%, 90%, or 95%) of the liver.

The present invention can be used to eliminate or reduce liver cancer stem cells. In one embodiment, the administration of one or more GEP inhibitors (such as, anti-GEP antibody) to a subject who had surgical resection of a primary liver tumor can be used to prevent the recurrence of liver tumor or cancer. In one embodiment, the administration of one or more of anti-GEP antibody or GEP antisense polynucelotide to a subject who had surgical resection of a primary liver tumor can be used to treat liver tumor or cancer.

In one embodiment, anti-GEP antibody GEP antisense polynucleotide may be administered to a subject who had partial hepatectomy or liver transplantation. In one embodiment, the present invention can be used to prevent or reduce the likelihood of liver tumor or cancer recurrence.

In one embodiment, the subject has undergone surgical resection of an entire primary liver tumor. In one embodiment, the subject has undergone surgical resection of part of a primary liver tumor.

In one embodiment, anti-GEP antibody or GEP antisense polynucleotide may be administered before or after the subject receives surgical resection of the liver tumor.

GEP Inhibitors

The anti-GEP antibody may bind specifically to human GEP.

Embodiments of anti-GEP antibodies are also described in WO2008/064570.

In accordance with the anti-GEP antibody and NK cells and/or an agent that promotes natural killer (NK) cell activity the invention may be administered to a subject that has liver cancer as a composition.

In certain embodiments, the anti-GEP antibody may bind specifically to a GEP protein of non-human animal species including, but not limited to, dogs, cats, horses, pigs, sheep, goats, chickens, mice, rats, and guinea pigs. The skilled artisan can readily make antibodies, aptamers, or binding partners that specifically bind to GEP proteins that are publically known. Described herein is a fusion construct comprising the antibody that binds specifically to a GEP protein (such as human GEP).

In one embodiment, GEP-specific antibodies can be generated by immunizing BALB/c mice or New Zealand white rabbits with GEP specific peptide sequences, as described in WO2008/064570 .

"Specific binding" or "specificity" refers to the ability of a protein to detectably bind an epitope presented on a protein or polypeptide molecule of interest, while having relatively little detectable reactivity with other proteins or structures. Specificity can be relatively determined by binding or competitive binding assays, using, *e.g.*, Biacore instruments. Specificity can be exhibited by, *e.g.,* an about 10:1, about 20:1, about 50:1, about 100:1, 10.000:1 or greater ratio of affinity/avidity in binding to the specific target molecule versus nonspecific binding to other irrelevant molecules.

Anti-GEP antibodies for the use as claimed may be in any of a variety of forms, including intact immunoglobulin molecules, fragments of immunoglobulin molecules such as Fv, Fab and similar fragments; multimers of immunoglobulin molecules (e.g., diabodies, triabodies, and bispecific and tri-specific antibodies, as are known in the art; *see, e.g.,* Hudson and Kortt, J. Immunol. Methods 231:177 189, 1999); fusion constructs containing an antibody or antibody fragment; and human or humanized immunoglobulin molecules or fragments thereof.

Antibodies for the use as claimed may be of any isotype, including IgG, IgA, IgE, IgD, and IgM. IgG isotype antibodies can be further subdivided into IgG1, IgG2, IgG3, and IgG4 subtypes. IgA antibodies can be further subdivided into IgA1 and IgA2 subtypes.

Antibodies for the use as claimed may include polyclonal and monclonal antibodies. The term "monoclonal antibody," as used herein, refers to an antibody or antibody fragment obtained from a substantially homogeneous population of antibodies or antibody fragments (*i.e.* the individual antibodies within the population are identical except for possible naturally occurring mutations that may be present in a small subset of the antibody molecules).

A monoclonal antibody composition is typically composed of antibodies produced by clones of a single cell called a hybridoma that secretes (produces) only one type of antibody molecule. The hybridoma cell is formed by fusing an antibody-producing cell and a myeloma or other self-perpetuating cell line. Such antibodies were first described by Kohler and Milstein, Nature, 1975, 256:495-497. An exemplary hybridoma technology is described by Niman et al., Proc. Natl. Acad. Sci. U.S.A., 1983, 80:4949-4953. Other methods of producing monoclonal antibodies, a hybridoma cell, or a hybridoma cell culture are also well known. See *e.g.,* Antibodies: A Laboratory Manual, Harlow et $\alpha$l., Cold Spring Harbor Laboratory, 1988; or the method of isolating monoclonal antibodies from an immunological repertoise as described by Sasatry, et al., Proc. Natl. Acad. Sci. USA, 1989, 86:5728-5732; and Huse et al., Science, 1981, 246:1275-1281.

The GEP inhibitor antisense polynucleotide preferably targets human GEP mRNA.

In some embodiments, the GEP antisense polynucleotide targets GEP mRNAs of non-human animals including, but not limited to, dogs, cats, horses, pigs, sheep, goats, chickens, mice, rats, and guinea pigs. The skilled artisan would readily appreciate that the antisense polynucleotides can be designed to target any GEP mRNAs publically known.

In some embodiments, the GEP inhibitor is a siRNA having a sequence sufficiently complementary to a target GEP mRNA sequence to direct target-specific RNA interference (RNAi).

In some embodiments, the GEP inhibitor is siRNA having a sequence sufficiently complementary to a target human GEP mRNA sequence to directtarget-specific RNA interference.

Examples of antisense polynucleotides include, but are not limited to, single-stranded DNAs and RNAs that bind to complementary target GEP mRNA and inhibit translation and/or induce RNaseH-mediated degradation of the target transcript; siRNA oligonucleotides that target or mediate GEP mRNA degradation; ribozymes that cleave GEP mRNA transcripts; and nucleic acid aptamers and decoys, which are non-naturally occurring oligonucleotides that bind to and block GEP protein targets in a manner analogous to small molecule drugs.

The term "nucleotide" refers to a nucleoside having one or more phosphate groups joined in ester linkages to the sugar moiety. Exemplary nucleotides include nucleoside monophosphates, diphosphates and triphosphates. The terms "polynucleotide" and "nucleic acid molecule" are used interchangeably herein and refer to a polymer of nucleotides joined together by a phosphodiester linkage between 5' and 3' carbon atoms. The terms "nucleic acid" or "nucleic acid sequence" encompass an oligonucleotide, nucleotide, polynucleotide, or a fragment of any of these, DNA or RNA of genomic or synthetic origin, which may be single-stranded or double-stranded and may represent a sense or antisense strand, peptide nucleic acid (PNA), or any DNA-like or RNA-like material, natural or synthetic in origin. As will be understood by those of skill in the art, when the nucleic acid is RNA, the deoxynucleotides A, G, C, and T are replaced by ribonucleotides A, G, C, and U, respectively.

As used herein, the term "RNA" or "RNA molecule" or "ribonucleic acid molecule" refers generally to a polymer of ribonucleotides. The term "DNA" or "DNA molecule" or deoxyribonucleic acid molecule" refers generally to a polymer of deoxyribonucleotides. DNA and RNA molecules can be synthesized naturally (e.g., by DNA replication or transcription of DNA, respectively). RNA molecules can be post-transcriptionally modified. DNA and RNA molecules can also be chemically synthesized. DNA and RNA molecules can be single-stranded (*i.e.,* ssRNA and ssDNA, respectively) or multi-stranded (*e.g.,* double stranded, *i.e.,* dsRNA and dsDNA, respectively). Based on the nature of the invention, however, the term "RNA" or "RNA molecule" or "ribonucleic acid molecule" can also refer to a polymer comprising primarily (*i.e.,* greater than 80% or, preferably greater than 90%) ribonucleotides but optionally including at least one nonribonucleotide molecule, for example, at least one deoxyribonucleotide and/or at least one nucleotide analog.

As used herein, the term "nucleotide analog," also referred to herein as an "altered nucleotide" or "modified nucleotide," refers to a non-standard nucleotide, including non- naturally occurring ribonucleotides or deoxyribonucleotides. Preferred nucleotide analogs are modified at any position so as to alter certain chemical properties of the nucleotide yet retain the ability of the nucleotide analog to perform its intended function.

As used herein, the term "RNA interference" ("RNAi") refers to a selective intracellular degradation of RNA. RNAi occurs in cells naturally to remove foreign RNAs (e.g., viral RNAs). Natural RNAi proceeds via fragments cleaved from free dsRNA which direct the degradative mechanism to other similar RNA sequences. Alternatively, RNAi can be initiated by the hand of man, for example, to silence the expression of endogenous target genes.

As used herein, the term "small interfering RNA" ("siRNA") (also referred to in the art as "short interfering RNAs") refers to an RNA (or RNA analog) comprising between about 10-50 nucleotides (or nucleotide analogs) which is capable of directing or mediating RNA interference.

As used herein, a siRNA having a "sequence sufficiently complementary to a target mRNA sequence to direct target-specific RNA interference (RNAi)" means that the siRNA has a sequence sufficient to trigger the destruction of the target mRNA (e.g., GEP mRNA) by the RNAi machinery or process. "mRNA" or "messenger RNA" or "transcript" is single-stranded RNA that specifies the amino acid sequence of one or more polypeptides. This information is translated during protein synthesis when ribosomes bind to the mRNA.

The present invention also contemplates vectors (e.g., viral vectors) and expression constructs comprising the nucleic acid molecules useful for inhibiting GEP expression and/or activity. In an embodiment, the vector comprises a siRNA that targets GEP mRNA. In another embodiment, the vector comprises a nucleic acid molecule encoding an anti-GEP A antibody.

As used herein, the term "expression construct" refers to a combination of nucleic acid sequences that provides for transcription of an operably linked nucleic acid sequence. As used herein, the term "operably linked" refers to a juxtaposition of the components described, wherein the components are in a relationship that permits them to function in their intended manner. In general, operably linked components are in contiguous relation.

Expression constructs of the invention will also generally include regulatory elements that are functional in the intended host cell in which the expression construct is to be expressed. Thus, a person of ordinary skill in the art can select regulatory elements for use in, for example, bacterial host cells, yeast host cells, mammalian host cells, and human host cells. Regulatory elements include promoters, transcription termination sequences, translation termination sequences, enhancers, and polyadenylation elements.

An expression construct as described herein can comprise a promoter sequence operably linked to a polynucleotide sequence encoding a peptide of the invention. Promoters can be incorporated into a polynucleotide using standard techniques known in the art. Multiple copies of promoters or multiple promoters can be used in an expression construct.

A promoter may be positioned about the same distance from the transcription start site as it is from the transcription start site in its natural genetic environment. Some variation in this distance is permitted without substantial decrease in promoter activity. A transcription start site is typically included in the expression construct.

Agents That Promote NK Cell Activity

Agents that promote NK cell activity include, IL-15, IL-12, pectin, interferons (such as IFN-gamma), IL-2, and IL-21.

Interleukin-12 (IL-12) is a cytokine that promotes and stimulates activity of NK cells and T lymphocytes, and has antitumor activity (U.S. Patent Application Publication No. 2012/0251441).

Interleukin 15 (IL-15) is a cytokine that stimulates NK cells [Fehniger T A, Caligiuri M A. Blood 97(1):14-32 (2001)]. It has become apparent that IL-15 presented through cell-to-cell contact has a higher NK stimulating activity than soluble IL-15 [Dubois S, et al., Immunity 17(5):537-547 (2002); Kobayashi H, et al., Blood (2004) PMID: 15367431; Koka R, et al., J Immunol 173(6):3594-3598 (2004); Burkett P R, et al., J Exp Med 200(7):825-834 (2004)]. (U.S. Patent Application Publication No. 2012/0015434).

Pectin is generally a natural macromolecular polysaccharide polymer, widely distributed in plant cell walls (Hyunjo Kim, et al. International Journal of Pharmaceutics, 1998, 161:149-159). Pectin can improve the immune function of hosts by enhancing the mononuclear phagocyte system, activating macrophages, T cells, B cells, NK cells and complement system; promoting cytokine secretion; enhancing immunity of erythrocytes; and exerting direct anticancer effects by changing the growth characteristics of solid cancer cell membrane, affecting signal transmission path in solid cancer cells and anti-free radical effect, inducing differentiation and apoptosis, inhibiting synthesis of nucleic acid and protein of the solid cancer cells, affecting ultrastructure of the solid cancer cells, affecting oncogenes and antimutation effect, and inhibiting vascularization of solid cancer (Chinese Journal of Information on Traditional Chinese Medicine, 1999, 5:64) (U.S. Patent Application Publication No. 2012/0244193).

Interleukin 21 (IL-21) promotes the expansion and maturation of NK cells. (U.S. Patent Application Publication No. 2011/0268766).

Interferons (such as, IFN-gamma) and IL-2 have been shown to promote NK cell activity.

Agents that increase MICA expression on a liver cancer cell.

Agents that promote MICA expression on a liver cancer cell include, but are not limited to, agents which inhibit the expression or activity of GEP, for example as described herein.

Therapeutic Compositions and Routes of Administration

Described herein are therapeutic or pharmaceutical compositions.

A composition may comprise the agents as set forth in the claims and, optionally, a pharmaceutically acceptable carrier.

The therapeutic composition can be any form of pharmaceutical format, including injectable formulations such as liquid and lyophilized injections.

Suitable non-toxic pharmaceutically acceptable carriers for use with the agent will be apparent to those skilled in the art of pharmaceutical formulation. See, for example, Remington's Pharmaceutical Sciences, seventeenth edition, ed. Alfonso R. Gennaro, Mack Publishing Company, Easton, Pa. (1985). Suitable carriers include ethanol, dimethyl sulfoxide, glycerol, silica, alumina, starch, sorbitol, inosital, xylitol, D-xylose, manniol, powdered cellulose, microcrystalline cellulose, talc, colloidal silicon dioxide, calcium carbonate, magnesium carbonate, calcium phosphate, calcium aluminum silicate, aluminum hydroxide, sodium starch phosphate, lecithin, and equivalent carriers and diluents. Saline solutions and aqueous dextrose and glycerol solutions can also be employed as liquid carriers, particularly for injectable solutions.

Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The therapeutic composition, if desired, can also contain minor amounts of wetting or emulsifying agents, or pH buffering agents.

The amount of active ingredient that may be combined with the carrier materials to produce a single dosage form will vary, depending on the type of the condition and the subject to be treated. In general, a therapeutic composition contains from about 5% to about 95% active ingredient (w/w). More specifically, a therapeutic composition contains from about 20% (w/w) to about 80%, or about 30% to about 70%, active ingredient (w/w).

The preparation of a pharmacological composition that contains active ingredients dissolved or dispersed therein is well understood in the art and need not be limited based on formulation. Typically such compositions are prepared as injectables either as liquid solutions or suspensions; however, solid forms suitable for solution, or suspensions, in liquid prior to use also can be prepared. The preparation also can be emulsified.

The therapeutic composition can include pharmaceutically acceptable salts of the components therein. Pharmaceutically acceptable salts include the acid addition salts (formed with the free amino groups of a polypeptide) that are formed with inorganic acids such as, for example, hydrochloric or phosphoric acids, or such organic acids as acetic, tartaric, mandelic and the like. Salts formed with the free carboxyl groups also can be derived from inorganic bases such as, for example, sodium, potassium, ammonium, calcium or ferric hydroxides, and such organic bases as isopropylamine, trimethylamine, 2-ethylamino ethanol, histidine, procaine and the like.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal.

Described herein is a pharmaceutical pack or kit comprising one or more containers filled with one or more of the ingredients, e.g., compound, carrier suitable for administration.

The compositions can be administered to the subject being treated by standard routes, including oral, inhalation, or parenteral administration including intravenous, subcutaneous, topical, transdermal, intradermal, transmucosal, intraperitoneal, intramuscular, intracapsular, intraorbital, intracardiac, transtracheal, subcutaneous, subcuticular, intraarticular, subcapsular, subarachnoid, intraspinal, epidural and intrasternal injection, infusion, and electroporation, as well as co-administration as a component of any medical device or object to be inserted (temporarily or permanently) into a subject.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi.

Sterile injectable solutions are prepared by incorporating the active ingredients in the required amount in the appropriate solvent followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and freeze-drying techniques, which yield a powder of the active ingredient, plus any additional desired ingredient from a previously sterile-filtered solution thereof.

Soluble GEP and soluble MICA as biomarkers for liver cancer

Described herein is a method of determining the suitability of a subject with liver cancer for treatment with an agent that inhibits the expression or activity of granulin-epithelin precursor (GEP), the method comprising i) obtaining a sample from the subject and ii) measuring the amount of soluble MICA in the sample.

The method may further comprise comparing the amount of soluble MICA in the sample to a chart correlating a quantity of soluble MICA and the presence or progression of liver cancer, wherein an amount of soluble MICA in the sample which correlates with presence or progression of liver cancer indicates suitability of the subject for treatment with an agent that inhibits the expression or activity of granulin-epithelin precursor (GEP).

Also described herein is a method of assessing or monitoring the extent or progression of liver cancer in a subject, the method comprising analysing a sample from the subject for a quantity of soluble GEP.

The method may be performed *in vitro,* on a sample removed from the subject. Preferably, the sample is a circulatory sample, i.e. a blood sample, a plasma sample or a serum sample.

The method may comprise a binding assay, comprising i) contacting a sample from a subject with a support; ii) contacting the sample with a detection binding partner which is specific for soluble GEP or soluble MICA; and iii) detecting the fraction of detecting binding partner bound to the support. Optionally, the method may comprise washing the support after incubation with a detection binding partner to remove any unbound or non-specifically bound binding partner. A detection binding partner may be linked to a signal (e.g. an enzyme) or can itself be detected by a secondary antibody that is linked to a signal for example through bioconjugation (e.g. in an indirect immunoassay format).

The method may comprise the step of developing and/or measuring the signal.

The method described may be any suitable method for determining a quantity of a soluble GEP or MICA in a sample. The method may be qualitative or quantitative. Suitable methods include a binding assay, for example an immunoassay, such as an ELISA, a Western blot, or an RIA assay, spectrometric methods for example mass spectrometry, nuclear magnetic resonance spectrometry, and combinations thereof; mass spectrometry assays coupled to immunaffinity assays; matrix-assisted laser desorption/ionization time-of-flight (MALDI-TOF) mass mapping and liquid chromatography/quadrupole time-of-flight electrospray ionization tandem mass spectrometry (LC/Q-TOF-ESI-MS/MS) sequence tag of proteins separated by twodimensional polyacrylamide gel electrophoresis (2D-PAGE) (Kiernan et al., Anal. Biochem., 301: 49-56, 2002; Poutanen et al., Mass Spectrom., 15: 1685-1692, 2001); quantitative mass spectroscopic methods, such as SELDI; MRM mass spectrometry or tandem mass spectrometry (such as LC-MRM-MS); flow cytometry; and immunohistochemical techniques (see for example Ausubel et al. (Current Protocols in Molecular Biology, John Wiley & Sons, New York, 1998).

The assay may be based upon measurement of direct signal (e.g. where signal is not amplified, such as coloured particles or fluorescence based assays) or measurement of generated signal, e.g. where signal is developed and/or amplified, for example by a catalyst or enzyme. The assay is preferably an ELISA assay, for example a direct, sandwich, indirect or competition ELISA. Such an assay preferably uses a binding partner for GEP, such as an anti-GEP antibody, for example a monoclonal antibody or polyclonal antibody, preferably A23 as described herein.

The signal may be an enzyme. Similar formats of assay are also possible where the signal is not an enzyme. Variations and formats of immunoassays and ELISA assays are possible where appropriate, and are known in the art.

The method may comprise the step of comparison to a control sample. This may be particularly relevant for quantitative assays. Where a qualitative result is suitable, comparison to a control may not be required. A control is a sample or standard used for comparison with a test sample. It may be derived from a healthy patient, such as one that does not have liver cancer. It will be appreciated that various different forms of controls may be used in such methods, depending upon the condition being assessed. A control sample may be an average value, obtained from a plurality of control subjects as defined above, and represented on a chart. A control can also be represented by a reference value or range of values representing an amount of activity or expression determined to be representative of a given condition. Reference values can include a range of values, real or relative expected to occur under certain conditions. These values can be compared with experimental values to determine if a biomarker is up-regulated or down-regulated in a particular sample for instance.

A quantity indicative of liver cancer or suitability for treatment with an agent which inhibits the activity or expression of GEP may be elevated levels of soluble GEP or soluble MICA compared to levels in a control sample or compared to a reference value, or within a margin of 20%, 15%, 10%, 5% or less. By "elevated" may mean at least 1.5 fold, at least 2-fold, at least 3-fold, at least 4-fold, at least 8-fold, or greater increase or decrease compared to a normal subject or a control.

The method may be qualitative, providing a binary positive/negative indication, or may be quantitative. In the latter embodiment, the quantity may be indicated by the strength of signal provided in a Western blot or immunoassay including RIA and ELISA. Signal intensity in an immunoassay may be measured, for example, by determining the absorbance or reflection of a light, including fluorescent signals passed through the sample. Preferably, a step of quantification may be performed prior to comparison with a control sample or reference value.

The method may further comprise providing a result to the subject, a clinician or other caregiver. An output may show a cut-off value or level that indicates presence or progression of liver cancer. The output may be a paper output (for example, a written or printed output), a display on a screen, a graphical output (for example, a graph, chart, voltammetric trace, or other diagram), or an audible output. The output may be communicated to the user, for example by providing an output via physical, audible, or electronic means (for example by mail, telephone, facsimile transmission, email, or communication to an electronic medical record). The output may be a diagnosis, prognosis or an indication that further tests may be required. The output may provide a recommended therapeutic regimen. Preferably, the output is adjusted to account for any background signal which may be measured prior to, or during the assay.

The calculated result can then be transmitted to a display device, which will present the signal is a readable format. This may be a yes/no type result, in the form of words or signs, or may be a quantitative result providing a value which is indicative of the amount of analyte present. Alternatively a result decision and raw data may be transmitted by wired, wireless far field or wireless near field communication techniques to a receiving "reader" docking device. A reader would be capable of relaying the information to a computer or through a computer network to a remote computer or to a hand held computing device (e.g. smart phone or tablet computer). Such a computing device could provide electronic storage and also permit more detailed analysis such as but not limited to trend analysis. The results could also be made available to a remote clinician.

In some examples, the methods further include a care pathway involving further diagnostic tests, treatment or preventative therapy, for example if the subject is diagnosed as being suitable for treatment with an agent which inhibits activity or expression of GEP.

Thus, a method described herein may further comprise, depending on the subject's results, a) additional diagnostic tests; b) prescribing a treatment regimen for the subject (e.g an agent which inhibits activity or expression of GEP or increases MICA on cancer cells); c) not prescribing a treatment regimen for the subject; or d) administering a treatment (such as surgery, chemotherapy, radiotherapy, to the subject if the subject's determined diagnosis is positive; or e) further monitoring using a method of the invention after a suitable time interval. The treatment regime may be any suitable treatment known to be suitable for preventing or reducing the cause and/or symptoms associated with the liver cancer.

Materials and Methods

Cell culture and assays

Human HCC cell lines, Hep3B and HepG2, were purchased from American Type Culture Collection (Manassas, VA) and were cultured; while Huh7 was purchased from Health Science Research Resources Bank (Osaka, Japan) and maintained in Dulbecco's modified Eagle medium (DMEM) supplemented with 10% heat-inactivated fetal bovine serum (Invitrogen, Carlsbad, CA).

Stable transfectant for GEP over-expression was established by transfecting GEP full-length cDNA into HepG2, a cell line with low endogenous GEP level; while GEP suppression was performed by transfecting GEP shRNA into Hep3B, a cell line with high endogenous GEP level. Both GEP over-expression and suppression transfectants were maintained in 10% AMEM with 0.4 mg/ml G418.

GEP blockage in Hep3B was performed by incubating the cells with or without 50 μg/ml anti-GEP monoclonal antibody A23 or mouse IgG isotype control antibody (Sigma-Aldrich, St. Louis, MO) for 24 h. GEP stimulation in HepG2 cells was performed by incubating the cells with or without 0.8 μg/ml recombinant GEP for 24 h.

Clinical specimens

The study protocol was approved by the Institutional Review Board of the University of Hong Kong/Hospital Authority Hong Kong West Cluster (HKU/HA HKW IRB). Five patients who underwent curative partial hepatectomy or liver transplantation for HCC at Queen Mary Hospital, Hong Kong, were recruited with written informed consent to the study. The patients had been diagnosed with primary HCC and confirmed by pathological examinations. Ethics approval for the use of archived fetal tissues identified from a computer database has been obtained from the Institutional Review Board of the University of Hong Kong/Hospital Authority Hong Kong West Cluster (HKU/HA HKW IRB).

Immunofluorescence staining and flow cytometric analysis

For the surface expression of MICA and HLA-E, cells were stained with APC-conjugated mouse anti-human MICA, PE-conjugated mouse anti-human HLA-E antibodies or equal amount of corresponding isotype control antibodies (BD Biosciences).

For co-expression with GEP, cells were stained with the above surface markers, followed by permeabilization with ice-cold 0.1% saponin and then incubated with FITC-conjugated mouse anti-human GEP antibody (homemade, described previously) or equal amount of FITC- conjugated mouse IgG isotype antibody (Sigma).

Preparation of human effector cells

Human peripheral blood mononuclear cells (PBMC) were freshly isolated from the huffy coats of healthy donors by density gradient centrifugation using Ficoll-Paque Plus. NK cells were isolated from PBMC using magnetic cell sorting by positive selection with anti-CD56 microbeads, while the unlabeled flow-through were the NK cell-depleted PBMCs, according to the manufacturer's instructions (Miltenyi Biotech). The resulting PBMCs, NK cells, and NK cell-depleted PBMCs were washed with RPMI 1640 with 10% FBS and used as human effector cells in the cell cytotoxicity assay.

Cell cytotoxicity assay

Cell cytotoxicity was determined by dual-color flow cytometry using the HCC cell lines Hep3B or HepG2 as target cells, while PBMC, NK cells, or NK cell-depleted PBMCs were used as effector cells. Briefly, HCC cells ($1 \times 10^6$ cells/ml) were labeled with the green fluorescence cytoplasmic dye 5- and (6)-carboxyfluorescein diacetate,succinimidylester (CSFE) at 0.1 $\mu$M for 15 min at 37°C in dark. After washing, CFSE-labeled HCC cells were then co-cultured with or without effector cells at indicated effector to target cells ratio (E/T ratio) in RPMI supplemented with 10% FBS for additional 5 hr at 37°C in 5% C02. Cells were then collected and stained with 10 $\mu$g/ml propidium iodide (PI) for 15 min at room temperature in dark. Cells were subject to dual-color flow cytometric analysis, with lysed target cells recognized as $CFSE^+PI^+$, while viable target cells as $CFSE^+PI^-$. Cell cytotoxicity level was expressed as percentage of lysed cells within the $CFSE^+$ target cells (Hep3B or HepG2), and calculated as follows:

$$\text{Percentage of } CFSE^+PI^+ \text{cells } I \text{ (Percentage of } CFSE^+PI^+ \text{ cells plus percentage of } CFSE^+PI^- \text{ cells) x 100.}$$

For blocking experiments, neutralization of MICA was performed by incubating the CFSE-labeled HCC cells with or without anti-MICA neutralizing antibody or mouse IgG2a isotype control antibodies (2 ug/ml, Biolegend) and co-cultured with effector cells.

For antibody-dependent cytotoxicity (ADCC), CFSE-labeled HCC cells were stained with or without indicated concentrations of anti-GEP antibody A23, or mouse IgG isotype antibody for 30 min at 4°C. Labeled target cells were then co-cultured with effector cells at indicated E/T ratio.

Statistical analyses

All data were expressed as mean values $\pm$ standard deviation (SD) from at least three independent experiments. Differences between groups were assessed by the Student's t test. A probability (p) < 0.05 was considered significantly different. All analyses were performed using the statistical software GraphPad Prism for Windows, Version 3.00 (Graph-Pad Software, CA, USA).

EXAMPLES

Following are examples that illustrate procedures for practicing the invention. These examples should not be construed as limiting.

EXAMPLE 1 - GEP EXPRESSION OF HCC CELLS MODULATES ANTI-TUMOR CYTOTOXIC ACTIVITY OF IMMUNE CELLS

It is postulated that only a restricted minority of tumorigenic malignant cells may possess the characteristics needed to modulate tumor-directed immune activation. To examine the effect of GEP on anti-tumor immunity, stable transfectant of GEP suppression was established using shRNA in Hep3B, a HCC cell line with high endogenous GEP; stable transfectant of GEP over-expression was established using GEP full-length cDNA in HepG2, a HCC cell line with low endogenous GEP level (Fig. 1A).

Cytotoxic activity of human PBMC against the HCC cells and their GEP transfectants was examined by dual-color flow cytometry. Cytotoxic activity of human PBMC against GEP-suppressed Hep3B cells was significantly higher than control Hep3B cells. On the contrary, cytotoxic activity of PBMC against HepG2 cells was significantly reduced when GEP was over-expressed.

The results show that GEP expression is crucial for rendering HCC cells resistant to antitumor cytotoxic activity of PBMC (Fig. 1B).

PBMC encompasses a variety of immune cells including T and B lymphocytes, monocytes, natural killer (NK) cells, and dendritic cells. The anti-tumor activity of NK cells in liver has been well documented. Clinical studies also suggest that NK cells contribute to innate defenses against primary liver tumors and liver metastases in patients.

To evaluate whether NK cells are involved in the GEP-modulated cytotoxic activity, NK cells were depleted from human PBMC by magnetic sorting. Upon NK cell depletion, the increased cytotoxic activity against GEP-suppressed Hep3B cells was abolished; while the reduction of cytotoxic activity against GEP over-expressed HepG2 was restored (Fig. 1C).

To further confirm the role of NK cells, HCC cells and their GEP transfectants were cocultured with NK cells at different E/T ratio. Cytotoxic activity of NK cells against GEP-suppressed Hep3B cells was significantly higher than that against control Hep3B cells; while significant reduction in NK cytotoxic activity was demonstrated in GEP over-expressed HepG2 cells when compared with control HepG2 cells (Fig. ID). The results show that NK cells play a role in the GEP-regulated anti-tumor cytotoxic activity.

EXAMPLE 2 - GEP DIFFERENTIALLY REGULATES THE EXPRESSION OF MICA AND HLA-E OF HCC CELLS

NK cell activity is regulated through the integrated signaling from stimulatory and inhibitory cell surface receptors that interact with ligands expressed on target cells. To elucidate the mechanism for GEP-regulated NK cell cytotoxicity, we determined the effect of GEP on the expression of ligands for activating immunoreceptor NKG2D and the stimulatory killer-cell immunoglobulin-like receptors (KIR), and inhibiting immunoreceptor CD94/NKG2A and inhibitory KIR2DL4 on HCC cells.

To delineate the effect of GEP, we measured the surface expression of MICA and HLA-E on Hep3B and HepG2 cells, and their GEP transfectants by flow cytometry. To further study the effect of GEP on the expression of MICA, we also analyzed the culture supernatants of the HCC cells and their GEP transfectants and measured the soluble MICA by ELISA.

Our results showed that upon GEP suppression, the expression of surface MICA significantly increased, while soluble MICA production and surface HLA-E expression significantly decreased when compared with control Hep3B cells, cell line with high endogenous GEP level (Fig. 2A). When GEP was over-expressed in HepG2, cell line with low endogenous GEP level, expression of surface MICA significantly decreased, while soluble MICA release and surface HLA-E level significantly increased when compared with control cells (Fig. 2B).

All these findings confirmed the regulatory role of GEP in the expression of MICA and HLA-E, rendering HCC cells resistant to NK cell cytotoxic activity.

EXAMPLE 3 - GEP BLOCKAGE BY ANTI-GEP ANTIBODY A23 MODULATES THE EXPRESSION OF MICA AND HLA-E OF HCC CELLS

To further validate the regulatory role of GEP on the expression of MICA and HLA-E, GEP blockage by anti-GEP monoclonal antibody A23 was performed in Hep3B cells. A23, but not mouse IgG, significantly suppressed GEP expression of Hep3B cells (Fig. 3A), demonstrating the suppressing effect was specific. A23 was found to significantly increase the expression of surface MICA, while decrease soluble MICA release and surface HLA-E expression of Hep3B when compared with control and isotype control treatment (Fig. 3B).

EXAMPLE 4 - GEP BLOCKAGE BY ANTI-GEP ANTIBODY A23 ENHANCES NK CELL CYTOTOXICITY AGAINST HCC CELLS VIA MICA

Confirming the role of GEP in tumor immune evasion, we proceeded to study the therapeutic potential of anti-GEP monoclonal antibody A23 in anti-tumor immunity in HCC.

HCC cells were treated with A23 or isotype control antibody for 24h prior to co-culture with PBMC to assess the effect of A23 on anti-tumor cytotoxic activity against HCC cells. Results showed that cytotoxic acitivties of PBMC against A23-treated Hep3B and HepG2 cells were significantly higher than those of control and isotype control antibody-treated cells (Fig. 4A).

To confirm the role of MICA in A23-induced NK cell cytotoxicity against HCC cells, blocking antibody of MICA was used. Hep3B and HepG2 cells were treated with A23 for 24h, and then incubated with anti-MICA neutralizing antibody or isotype control antibody and co- cultured with PBMC. Results showed that the addition of anti-MICA blocking antibody could markedly abrogate the A23-induced cytotoxic activity against Hep3B and HepG2 cells, whereas there was no effect exerted by the mouse $IgG_{2a}$ isotype control antibody. The results suggested that the A23-induced cytotoxic activity against HCC cells was dependent on MICA. (Fig. 4A)

Since MICA-NKG2D signal is an important inhibitory signal to NK cells, we proceeded to study the effect of MICA neutralization on A23-induced NK sensitivity of Hep3B and HepG2 cells. Results showed that blocking MICA could significantly abolish the A23-induced NK sensitivity of HCC cells, therefore confirming that A23-induced NK cell cytotoxicity against HCC cells was dependent on the MICA-NKG2D (Fig. 4B).

EXAMPLE 5 - CO-EXPRESSION OF GEP WITH MICA AND HLA-E IN HCC CLINICAL SPECIMENS

The present inventors have shown that GEP conferred HCC cells resistance to NK cell cytotoxic activity by differentially regulated surface MICA and HLA-E expression in HCC cell line models. To confirm the immune evasion ability of GEP-expressing cells in clinical settings, GEP was co-stained with surface MICA and HLA-E in HCC clinical specimens by immunofluorescence staining and flow cytometry. Co-expression of GEP and MICA or HLA-E was performed by triple-color flow cytometry, gating on the albumin⁺ hepatocytes isolated from HCC tissues.

The results showed that GEP⁺ cells always expressed lower level of surface MICA than GEP⁻ cells (Fig. 5A). However, GEP was found to co-express with surface HLA-E in the HCC clinical specimens (Fig. 5B). The results are consistent with our cell line model that GEP down-regulated MICA, but up-regulated HLA-E on the surface of HCC cells, making the cells less susceptible to NK anti-tumor cytotoxic activity.

EXAMPLE 6 - ANTIBODY-DEPENDENT CELLULAR CYTOTOXICITY MEDIATED BY ANTI-GEP MONOCLONAL ANTIBODY A23

To further characterize the immunomodulatory mechanism of anti-GEP monoclonal antibody A23, antibody-dependent cellular cytotoxicity (ADCC) of A23 was assessed. ADCC occurs when antibodies bind to antigen on tumor cells and the antibody Fc domains engage Fc receptors on the surface of immune effector cells. Hep3B or HepG2 cells were stained with or without anti-GEP antibody A23 or mouse isotype control antibody for 30 min and the antibodylabeled cancer cells were then co-cultured with PBMC. A23, but not isotype control antibody, significantly induced ADCC of human PBMC against both Hep3B and HepG2 cells in a dosedependent manner (Fig. 6A). The results show that the anti-tumor activity of A23 was at least partly attributable to ADCC.

In addition, this Example further elucidates which effector cells are important for the ADCC activity of A23 in human. NK cells were depleted from PBMC and the A23-mediated ADCC in both cell lines was markedly abolished upon depletion of NK cells from PBMC (Fig. 6B). A23-labeled cancer cells were then co-cultured with NK cells and the A23-mediated ADCC against the HCC cells increased along the E/T ratio (Fig. 6C). Therefore, it was confirmed that NK cells also play an important role as effector cells for the A23-induced ADCC in human.

EXAMPLE 7 GEP AND IMMUNE ACTIVITIES OF HUMAN LIVER CANCER

Association of GEP and immune evasion ability in human liver cancer

Liver tissue specimens:

The expression levels and patterns of GEP and immune evasion related molecules in human liver cancer specimens are examined, including MICA (ligand for NK cell activating immunoreceptor) and HLA-E (ligand for NK cell inhibitory immunoreceptor). Transcript and protein expression levels of GEP, MICA and HLA-E are examined in tumor tissue and adjacent non-tumor liver tissues of liver cancer patients and in non-cancer liver tissues (including normal, hepatitis and cirrhosis) by real-time qPCR, immunohistochemical staining, and western blot analysis. The clinico-pathological features including survival outcomes with these molecules are analyzed. GEP modulated the expression of MICA and HLA-E, and the expression levels of GEP in patient specimens can be used to determine the role of GEP on immune evasion ability in clinical settings.

Blood specimens:

The levels of soluble MICA and GEP in the sera of liver cancer patients, patients with cirrhosis / hepatitis and healthy individuals are measured by enzyme-linked immunosorbent assay (ELISA). Since GEP expression induces shedding of MICA from HCC cells, soluble MICA and GEP levels in the serum sample of liver cancer patient can be used as a predictive marker for corresponding immunotherapy.

Association of GEP expression in human liver cancer and NK cell infiltration

Liver tissue specimens:

The GEP expression levels in liver cancer cells and the abundance of NK cell infiltration into the tumor bulk are investigated. The number of infiltrating NK cells is assessed in the tumor tissues and adjacent non-tumor liver tissues of liver cancer patients and in non-cancer liver tissues by immunohistochemical staining.

GEP expression rendered liver cancer cells resistance to NK cell cytotoxic activities. GEP antibody treatment enhanced the NK cell cytotoxic activities against liver cancer cells

Animal models:

The effect of GEP monoclonal antibody A23 treatment is examined in the human liver cancer xenografts in the mice models. To simulate the clinical situation of intervention on advanced liver cancer, treatment is started after large tumor bulk has been established in the mice. Mice receive GEP monoclonal antibody A23 or control treatment by intraperitoneal injection over extended time periods, and the xenografts are dissected and examined for transcript and protein expression of GEP, MICA and HLA-E by real-time qPCR and immunohistochemical staining. Infiltration of NK cells into the tumor bulk is examined by immunohistochemical staining. The role of GEP antibody on the tumor expressions of MICA and HLA-E, and the cytotoxic activities of NK cells in the tumor bulk is investigated.

Above, it has been demonstrated that granulin-epithelin precursor (GEP) protected liver cancer cells from cytotoxic activities of natural killer (NK) cells. These NK cells were isolated lymphocytes from healthy individuals. Using NK cells were isolated from liver cancer patients; it has been shown that these NK cells demonstrated cytotoxic activities against liver cancer cells (Figure 7). Thus, activation of NK cells in liver cancer patients, by GEP antibody or inhibitors as described herein, could result in control of tumor growth. Transfusion of NK cells to liver cancer patients would not be necessary.

## Claims

1. An anti-GEP antibody and (a) natural killer (NK) cells, or (b) an agent that promotes NK cell activity selected from IL-15, IL-12, pectin, interferon, IFN-gamma, IL-2 or IL-21, for use in treating liver cancer in a subject.

2. A GEP antisense polynucleotide and (a) natural killer (NK) cells, or (b) an agent that promotes NK cell activity selected from IL-15, IL-12, pectin, interferon, IFN-gamma, IL-2 or IL-21, for use in treating liver cancer in a subject.

3. An anti-GEP antibody for use in preventing recurrence of or treating liver cancer in a subject, wherein the subject has had surgical resection of a primary liver tumor, has undergone hepatectomy, and/or had a liver transplant; and wherein (a) an effective amount of NK cells is also administered; or (b) an agent that promotes NK cell activity selected from IL-15, IL-12, pectin, interferon, IFN-gamma, IL-2 or IL-21 is also administered.

4. A GEP antisense polynucleotide for use in preventing recurrence of or treating liver cancer in a subject, wherein the subject has had surgical resection of a primary liver tumor, has undergone hepatectomy, and/or has had a liver transplant; and wherein (a) an effective amount of NK cells is also administered; or (b) an agent that promotes NK cell activity selected from IL-15, IL-12, pectin, interferon, IFN-gamma, IL-2 or IL-21 is also administered.

5. The GEP antisense polynucleotide and (a) natural killer (NK) cells, or (b) an agent that promotes NK cell activity for the use as claimed in claim 2 or claim 4, wherein the GEP antisense polynucleotide is an siRNA.

## Patentansprüche

1. Anti-GEP-Antikörper und (a) natürliche Killerzellen (NK-Zellen) oder (b) eine Substanz, die eine NK-Zellaktivität fördert, ausgewählt aus IL-15, IL-12, Pektin, Interferon, IFN-Gamma, IL-2 oder IL-21, zur Verwendung in der Behandlung von Leberkrebs in einem Subjekt.

**2.** GEP-Antisense-Polynukleotid und (a) natürliche Killerzellen (NK-Zellen) oder (b) eine Substanz, die eine NK-Zellaktivität fördert, ausgewählt aus IL-15, IL-12, Pektin, Interferon, IFN-Gamma, IL-2 oder IL-21, zur Verwendung in der Behandlung von Leberkrebs in einem Subjekt.

**3.** Anti-GEP-Antikörper zur Verwendung in der Prävention des Wiederauftretens von oder der Behandlung von Leberkrebs in einem Subjekt, wobei an dem Subjekt eine chirurgische Resektion eines primären Lebertumors durchgeführt worden ist, es sich einer Hepatektomie unterzogen hat und/oder es eine Lebertransplantation erhalten hat; und wobei (a) eine wirksame Menge von NK-Zellen ebenfalls verabreicht wird; oder (b) eine Substanz, die eine NK-Zellaktivität fördert, ausgewählt aus IL-15, IL-12, Pektin, Interferon, IFN-Gamma, IL-2 oder IL-21, ebenfalls verabreicht wird.

**4.** GEP-Antisense-Polynukleotid zur Verwendung in der Prävention des Wiederauftretens von oder der Behandlung von Leberkrebs in einem Subjekt, wobei an dem Subjekt eine chirurgische Resektion eines primären Lebertumors durchgeführt worden ist, es sich einer Hepatektomie unterzogen hat und/oder es eine Lebertransplantation erhalten hat; und wobei (a) eine wirksame Menge von NK-Zellen ebenfalls verabreicht wird; oder (b) eine Substanz, die eine NK-Zellaktivität fördert, ausgewählt aus IL-15, IL-12, Pektin, Interferon, IFN-Gamma, IL-2 oder IL-21, ebenfalls verabreicht wird.

**5.** GEP-Antisense-Polynukleotid und (a) natürliche Killerzellen (NK-Zellen) oder (b) eine Substanz, die eine NK-Zellaktivität fördert, zur Verwendung nach Anspruch 2 oder Anspruch 4, wobei das GEP-Antisense-Polynukleotid siRNA ist.

**Revendications**

**1.** Anticorps anti-GEP et (a) cellules tueuses naturelles (NK), ou (b) agent qui favorise l'activité des cellules NK sélectionné parmi IL-15, IL-12, la pectine, l'interféron, l'IFN-gamma, IL-2 ou IL-21, pour utilisation dans le traitement du cancer du foie chez un sujet.

**2.** Polynucléotide antisens GEP et (a) cellules tueuses naturelles (NK), ou (b) agent qui favorise l'activité des cellules NK sélectionné parmi IL-15, IL-12, la pectine, l'interféron, l'IFN-gamma, IL-2 ou IL-21, pour utilisation dans le traitement du cancer du foie chez un sujet.

**3.** Anticorps anti-GEP pour utilisation dans la prévention d'une récurrence ou le traitement du cancer du foie chez un sujet, le sujet ayant eu une résection chirurgicale d'une tumeur du foie primaire, ayant subi une hépatectomie, et/ou ayant eu une greffe du foie ; et (a) une quantité efficace de cellules NK étant également administrée ; ou (b) un agent qui favorise l'activité des cellules NK sélectionné parmi IL-15, IL-12, la pectine, l'interféron, l'IFN-gamma, IL-2 ou IL-21 étant également administré.

**4.** Polynucléotide antisens GEP pour utilisation dans la prévention d'une récurrence ou le traitement du cancer du foie chez un sujet, le sujet ayant eu une résection chirurgicale d'une tumeur du foie primaire, ayant subi une hépatectomie, et/ou ayant eu une greffe du foie ; et (a) une quantité efficace de cellules NK étant également administrée ; ou (b) un agent qui favorise l'activité des cellules NK sélectionné parmi IL-15, IL-12, la pectine, l'interféron, l'IFN-gamma, IL-2 ou IL-21 étant également administré.

**5.** Polynucléotide antisens GEP et (a) cellules tueuses naturelles (NK), ou (b) agent qui favorise l'activité des cellules NK pour l'utilisation telle que revendiquée dans la revendication 2 ou la revendication 4, le polynucléotide antisens GEP étant un petit ARNi.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011140828 A
- WO 2008064570 A
- US 2008199470 A
- WO 2005009217 A
- WO 2005106019 A1

- US 20120251441
- US 20120015434
- US 20120244193
- US 20110268766

**Non-patent literature cited in the description**

- **HO, J. C. et al.** Granulin-epithelin precursor as a therapeutic target for hepatocellular carcinoma. *HEPATOLOGY,* vol. 47 (5), 1524-1532
- **MASAHISHA, J. et al.** Expression and role of MICA and MICB in human hepatocellular carcinomas and their regulation by retinoic acid. *Int. J. Cancer,* 2003, vol. 104, 354-361
- **KOHLER ; MILSTEIN.** *Nature,* 1975, vol. 256, 495-497
- **NIMAN et al.** *Proc. Natl. Acad. Sci. U.S.A.,* 1983, vol. 80, 4949-4953
- **HARLOW.** Antibodies: A Laboratory Manual. Cold Spring Harbor Laboratory, 1988
- **SASATRY et al.** *Proc. Natl. Acad. Sci. USA,* 1989, vol. 86, 5728-5732
- **HUSE et al.** *Science,* 1981, vol. 246, 1275-1281
- **FEHNIGER T A ; CALIGIURI M A.** *Blood,* 2001, vol. 97 (1), 14-32

- **DUBOIS S et al.** *Immunity,* 2002, vol. 17 (5), 537-547
- **KOBAYASHI H et al.** *Blood,* 2004
- **KOKA R et al.** *J Immunol,* 2004, vol. 173 (6), 3594-3598
- **BURKETT P R et al.** *J Exp Med,* 2004, vol. 200 (7), 825-834
- **HYUNJO KIM et al.** *International Journal of Pharmaceutics,* 1998, vol. 161, 149-159
- *Chinese Journal of Information on Traditional Chinese Medicine,* 1999, vol. 5, 64
- Remington's Pharmaceutical Sciences. Mack Publishing Company, 1985
- **KIERNAN et al.** *Anal. Biochem.,* 2002, vol. 301, 49-56
- **POUTANEN et al.** *Mass Spectrom.,* 2001, vol. 15, 1685-1692
- **AUSUBEL et al.** Current Protocols in Molecular Biology. John Wiley & Sons, 1998